# EUROPEAN PATENT SPECIFICATION

(11) **EP 0 784 996 B1**
(45) Date of publication and mention of the grant of the patent: **22.11.2006**
(21) Application number: 96850200.5
(22) Date of filing: 26.11.1996
(51) Int. Cl.: A61N 1/365

(54) **Device and method for generating a synthesized ECG**
Vorrichtung und Verfahren zur Erzeugung eines synthetisierten Elektrokardiogramms
Dispositif et méthode de génération d'un électrocardiogramme synthétique

(30) Priority: 28.11.1995 SE 9504258
(43) Date of publication of application: 23.07.1997
(73) Proprietor: St. Jude Medical AB, 175 84 Järfälla (SE)
(72) Inventor: Hirschberg, Jakub, 183 44 Täby (SE); Hedberg, Sven-Erik, 196 32 Kungsängen (SE)

(56) References cited:
- EP-A- 0 310 216
- EP-A- 0 443 496
- EP-A- 0 465 241
- EP-A- 0 596 319
- WO-A-92/02171
- US-A- 5 404 877

## Description

### The Field of the Invention

The invention relates to a device and a method for signal processing of known intracardiac signals from electrodes in an implantable heart stimulator/defibrillator/monitoring system (an active implanted system) and a method for use of detected intracorporal cardiac signals and also to an active implant including such a device in whole or in part.

### Description of Related Art

Today an ECG is recorded by applying electrodes (limb and chest electrodes) on the outside of the body at specific points and subsequently registering electrical activity in the heart Since the signals have to pass through tissue, muscles, bone and adipose tissue the signals are partly disguised and also the muscles in themselves display electrical activity which may hide or distort the signals from the heart.

A complementary measure to the ECG is to transmit signals by telemetry from intracardial electrodes in the form of an IEGM, an intracardiac electrogram. This may be done e.g. in follow-ups after implantation and when possibly adjustment of controlling parameters in the active implant occurs. The IEGM is usually combined by the practitioner with the measuring of a surface ECG.

Measuring of signals from implanted electrodes per se are known within the art. A method and an apparatus for detection of ECG and / or blood pressure (BP) waveforms and measuring of the R - R and Q - T intervals from the detected ECG signals so as to determine the pulse rate and to stimulate the heart is described in US,A,4 870 974.

The purpose of the method is to derive the VFT (Ventricular Fibrillation Threshold) indirectly from measurements of the PVCT (Premature Ventricular Contraction Threshold), MET (Multiple Extrasystole Threshold), and VTT (Ventricular Tachycardia Threshold). Stimulation pulses for the stimulation of the heart are given and also pulse trains intended to cause the above phenomena. The threshold is detected by incrementally giving the pulse trains a higher amplitude. However the ECG used in this publication is measured by extracorporal means, i.e., by surface measurements, while the stimulation pulses and the pulse trains are applied via either cardiac catheter or in the esophagus.

A method and an implantable pacemaker suited for Bradyarrhythmia therapy which stimulates and senses and obtains the ECG signal from the by stimulation evoked response in the heart, i.e. the QRS complex, is described in EP 605 244. The problem described therein is that of the large residual potential remaining across the bipolar electrodes. This problem is solved by processing the signals sensed by the ring and tip electrode of a typical pacemaker bipolar lead. The circuit senses each of these signals from the ring and tip electrodes in a unipolar mode. The sensed electrical signals are subsequently added to cancel or nullify the opposing residual potentials which remain on the ring and tip electrode post pacing. The common-potential ECG signal is added and presented for subsequent detection of the QRS complex. In this way the higher residual potential signals are removed prior to the subsequent signal processing and the low level ECG signal is retained. This is thus an adding procedure and not a processing of the signals in a differential mode.

Another example of combinations of signals and the use of time of incidence for acceptance of true atrial events by combining signals from different electrodes and using a logic unit is found in EP, A1,0 596 319 in which a heart stimulator is described comprising a pulse generator and an electrode system which contains at least one bipolar electrode with one electrode arranged in the atrium and one electrode in the ventricle respectively, for detection of atrial and ventricular activity. Document WO-A-92/02171 discloses an apparatus configured to synthesize ECG signals. This document is considered to represent the most relevant prior art.

However, none of the above mentioned patent documents mentions the possibility of creating an synthesized ECG, which gives information corresponding to the electrical activity of the heart in a form that resembles a surface ECG, but which is registered with electrodes which constitute part of an active implant.

### Summary of Terms used in this application

Active implant, heart stimulator, heart monitor, defibrillator and other similar implanted units.

SECG Synthesized surface electrocardiogram is a synthesized electrogram created from at least two IEGMs.

ECG Surface electrogram, a recording using limb and/or chest electrodes, i.e. extracorporal recordings.

IEGM Electrocardiogram from implanted , intracorporal electrode system.

TIP Tip electrode, pace and sensing electrode implanted in the ventricle or in the atrium.

RING Ring electrode, part of a bipolar electrode system. Can be implanted as a part of the ventricular and/or atrial electrode.

RV Right ventricular defibrillation electrode.

SVC Superior vena cava electrode, a defibrillation electrode place in superior vena cave.

PATCH Epicardial patch, subcutaneous patch, extracardial patch.

CAN Active implant housing.

Heart stimulator, pacemaker, defibrillator.

NN Neural Network

### Summary of the Invention

The object of the invention is to provide from the implanted electrodes of an active implant (pacer and/or a defibrillator and/or implanted monitoring systems) a type of ECG which in appearance can be substituted for a surface ECG and which may be used especially postoperatively and for follow-ups (repeated) after the implantation of a stimulator or the like or for e.g. checking the function of the same or for adjusting the parameters controlling the active implant or whenever a programmer is in contact with the active implant.

Another object is to provide easier and faster follow-ups after an implantation without the use of additional extracorporal electrodes.

Another object is to make possible the transmitting over the public telephone net of signals used by the practitioner for follow-ups and for diagnostic use by the practitioner located away from the patient having the active implant. Also the intent is to synthesize a surface ECG so that the practitioner will recognize the signals and be able to interpret the signals with his/her knowledge of the surface ECG.

Another object of the invention is to make use of the signals from each electrode and to be able to exclude and/or lessen the effects of the information in the signal of electrical activity in the very close proximity to the electrode.

Yet another object is to be able to better distinguish between paced and spontaneous heart activities such as: capture, inhibition, fusion beats, etc.

A further object is to provide a diagnosis means to be used in diagnosing and detection of ischemia and in resolving which type of tachycardia is present in tachycardia cases. The indications of ischemia in the heart shown by the implanted electrodes should be easier to detect and also to counteract via the pacing of the heart. This can be the earliest warning that can be seen of ischemia or other heart diseases.

Still another object is to find a means for registering of changes in the ECG in a person having an active implant, which changes will constitute contraindications or indications for adjustment of the rate responsive function in which case the rate will remain unchanged or adjusted in some other way indicated by analysis of the recorded ECG.

The inventors have now surprisingly found that by interconnecting two or more intracardiac and/or extracardiac electrodes - electrically connecting the electrodes - measuring signals from these electrodes and thereafter expose these signals to signal processing a synthesized surface ECG may be obtained. This signal processing may comprise a treatment of the signals in which the signals are processed using methods such as neural network and/or fuzzy logic, and/or summation of processed signals.

According to the invention it has been shown that IEGM recordings between different electrodes in defibrillation and pacing systems show similarities to "surface ECG". The idea arose to measure signals between at least two groups of interconnected electrodes, where each group contains signals from at least one electrode.

The problems solved according to the invention by using only in the heart implanted electrodes and/or implanted intra-corporal electrodes belonging to an active implant to create from measurements of electrical activity in the heart through signal processing a synthesized ECG which gives the same or other information as the standard surface ECG.

Another problem which is solved according to the invention is that the necessity for placing electrodes on the patients surface and connecting these to the ECG machine no longer will be necessary since the signals from the implanted electrodes may be sensed using telemetry and this gives the possibility to relay these signals even by telephone from the patient to the physician, which may make a printout of the signals and analyse the ECG.

These objects are achieved according to the invention defined in the independent claims.

Preferred embodiments of the invention are described in the dependent claims.

### Description of the Drawings

In order to explain the invention, reference is made below the figures of the drawings, wherein schematically is shown:
- Fig. 1: A schematic representation of an electrode configuration used in n active implant system (DDD,VDD) according to the prior art, using unipolar electrodes, which may be used in accordance with the invention.
- Fig. 2: A schematic representation of an electrode configuration in a bipolar system, which may be used in accordance with the invention.
- Fig. 3: A schematic representation of an electrode configuration in a defibrillation stimulator, which may be used in accordance with the invention.
- Fig. 4: Recordings of the typical signals from groups of two implanted electrodes and a surface ECG.
- Fig. 5: a) recordings of the signals measured between tip electrode in the right ventricle and the can, i.e. the stimulator case, before application of the transfer function.
b) the influence of a non-linear amplification (transfer function) on the same signals as in a).
- Fig. 6: Two IEGM recordings SVC-CAN, TIP-CAN.
- Fig. 7: A synthesized ECG from ((SVC-CAN) + (TIP-CAN)) and a surface ECG.
- Fig. 8: An enlargement of the diagram in Fig. 7.
- Fig. 9: Shows a) recordings of signal between two groups of unprocessed interconnected electrodes (tip + can) and (SVC superior vena cava and can)
- Fig. 10: A first embodiment of a heart stimulator system according to the invention.
- Fig. 11: A second embodiment of a heart stimulator according to the invention.
- Fig. 12: A third embodiment of a heart stimulator according to the invention.
- Fig. 13: A system according to the invention for registering and processing of signals from implanted electrodes.
- Fig. 14: An enlargement of the transforming unit, part of the system depicted in Fig. 13.
- Fig. 15: A schematic drawing of one possible implementation of a neural network for signal processing.
- Fig. 16: A first embodiment of a device according to the invention.
- Fig. 17: Two other embodiments of a device according to the invention.

### Description of Existing Electrode Configurations which may be used in accordance with the Invention

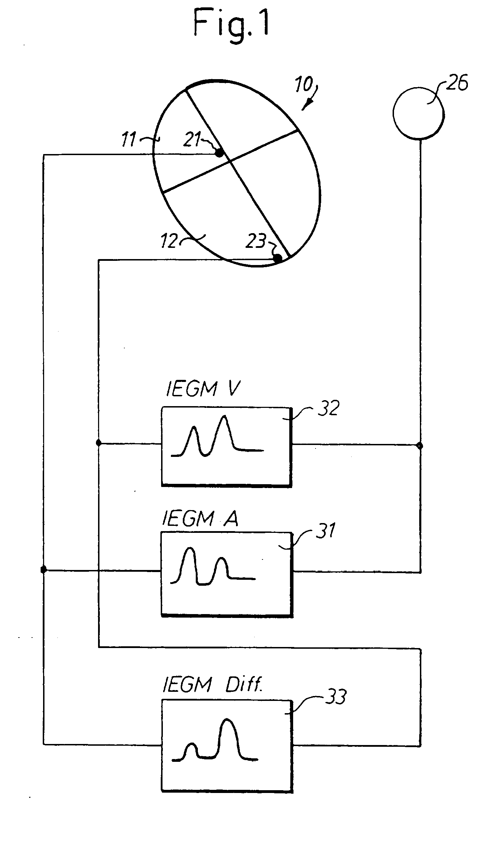

Fig. 1 shows schematically an existing electrode configuration for registering of IEGM (intracardiac electrogram) between different implanted electrodes. In the figure is depicted a heart 10 with two implanted unipolar electrodes 21, 23 in the atrium 11 and in the ventricle 12, respectively. Also shown is an extracardiac electrode 26, which may be the housing of the stimulator (can). Signals are measured between the can and electrodes 21, 23 giving an atrial IEGM A in unit (31) and a ventricular IEGM V unit (32). The differential signal between the two electrodes 21 and 23 gives a differential signal in unit (33). Units 31, 32, and 33 are the monitoring units for the IEGMs.

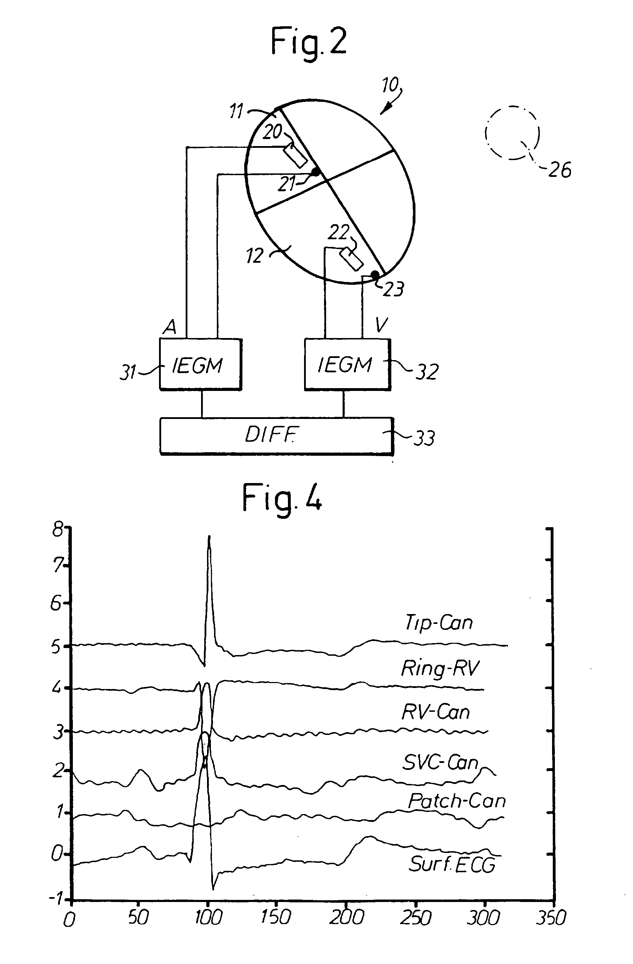

Fig. 2 shows another example of an existing electrode configuration which may be used in accordance with the invention in the figure is shown a heart 10 with two implanted bipolar electrodes, each having a tip electrode 21 and 23 and a ring electrode 20 and 22 in the atrium 11 and the ventricle 12, respectively. The atrial IEGM is then measured in the unit 31 between the ring and the tip of the bipolar electrode in the atrium 11 and the ventricular IEGM is measured in the unit 32 between the ring and the tip of the bipolar electrode in the ventricle 12. A differential IEGM can then be measured between the atrial and the ventricular IEGM:s in the unit 33. The can is indicated by the reference number 26.

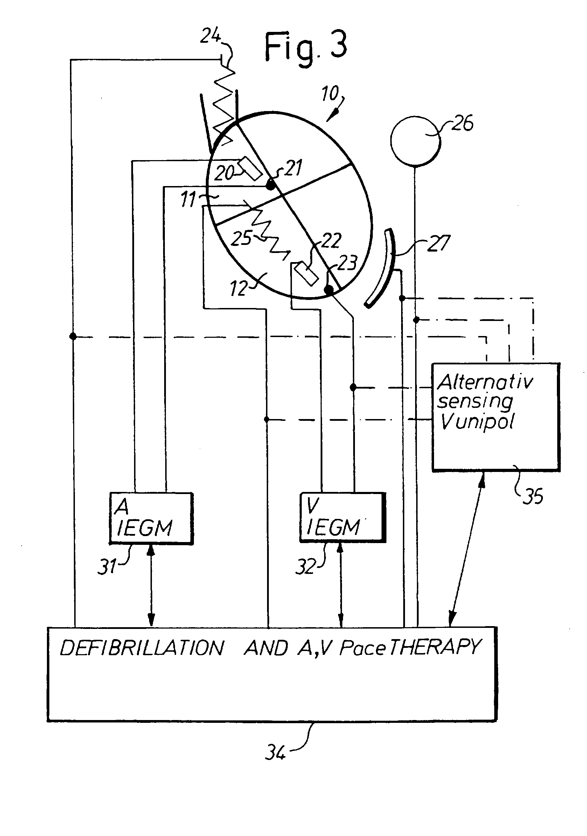

Fig. 3 shows an existing electrode configuration in a defibrillation system, which may be used in accordance with the invention. In the figure is shown a heart 10 with two implanted bipolar electrodes, each having a tip electrode 21, 23 and a ring electrode 20, 22 in the atrium 11 and the ventricle 12, respectively. There are also two defibrillation electrodes 24, 25 in the superior vena cava and the ventricle, respectively and a patch extracardial electrode 27 and the can 26, which also may be another extracardially implanted electrode. The atrial IEGM is then measured in the monitoring unit 31 between the ring 20 and the tip 21 of the bipolar electrode in the atrium 11 and the ventricular IEGM is measured in the monitoring unit 32 between the ring 22 and the tip 23 of the bipolar electrode in the ventricle 12. The registration is bipolar. Alternatively, so called unipolar sensing in the ventricle may be carried out between the patch 27 or the can 26 and tip electrode 23 or the defibrillation electrodes 24, 25 as indicated by 35. Alternatively, so called unipolar sensing in the ventricle may be carried out between tip 23 and electrodes 20, 24, 23, 26 or 27. Observe that semibipolar recording between the tip in the ventricle (23) and the tip in the atrium (21) is an another option. This system may be used for defibrillation and A, V pace therapy indicated by 34.

These examples shown in Fig. 1 - 3 are by no means exhaustive. Other electrode configurations may as well be used in these measurements

### Detailed Description and Preferred Embodiments.

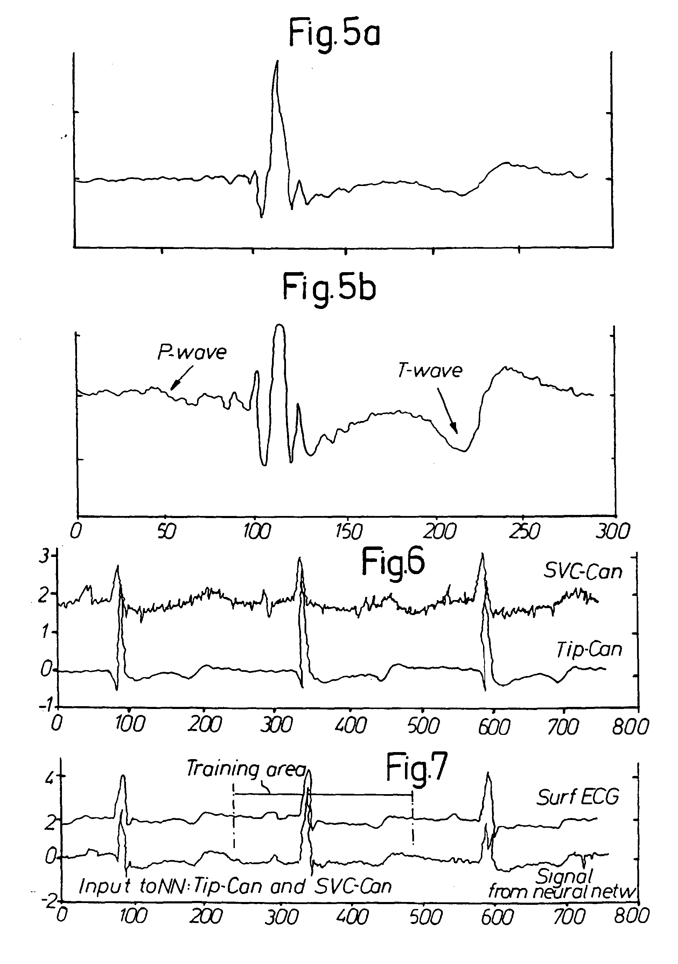

In order to explain the invention a discussion follows below as to the appearance of the signals registered between pairs of implanted intracardiac electrodes. Fig. 4 shows recordings of signals registered between:
a. Tip (V)- Can
b. Ring - RV (Defibrillation electrode right ventricle)
c. RV - Can
d. SVC (Superior Vena Cava) - Can
e. Patch - Can
f. A surface ECG (using limb leads)

The signals have been filtered (transformed) to partly remove effects from activities in the proximity of the electrodes.

From the figure can be seen that different characteristics from the different recorded IEGMs can be recognized in the surface ECG. The surface ECG thus is one form of a summation of the electrical activity in the heart. Of course the local phenomena at each electrode site will influence the signal registered by that electrode and prevail over influences from places further away from the actual electrode site.

Fig. 5 shows a) the recordings of the unprocessed signals registered between tip (V) and can and in b) the influence of non-linear amplification which lessens the influence from the local phenomena which one is not interested in this specific application according to the invention. This non-linear amplification will lead to smaller details becoming more visible in the signal, i.e. details that have no local character but are describing the total functioning of the heart. In this case the signals have been filtered/transformed or blocked in order to take away the local influence on the signals before the summation of the same. As may be noted the p-wave is emphasized by the non-linear amplification. The T-wave is also emphasized in the same manner as is indicated in the figure. The non-linear amplification, using a so called transfer function is discussed below.

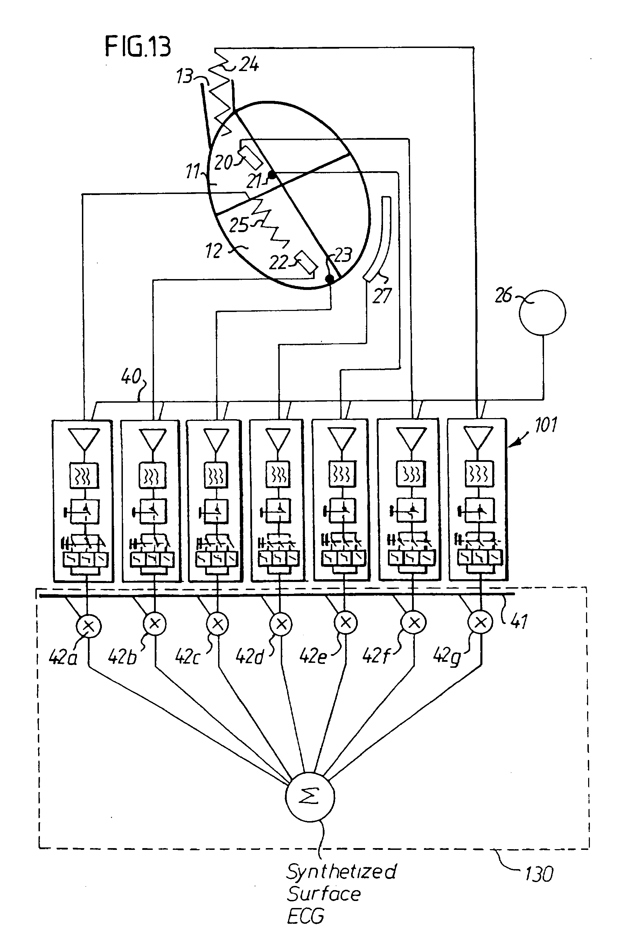

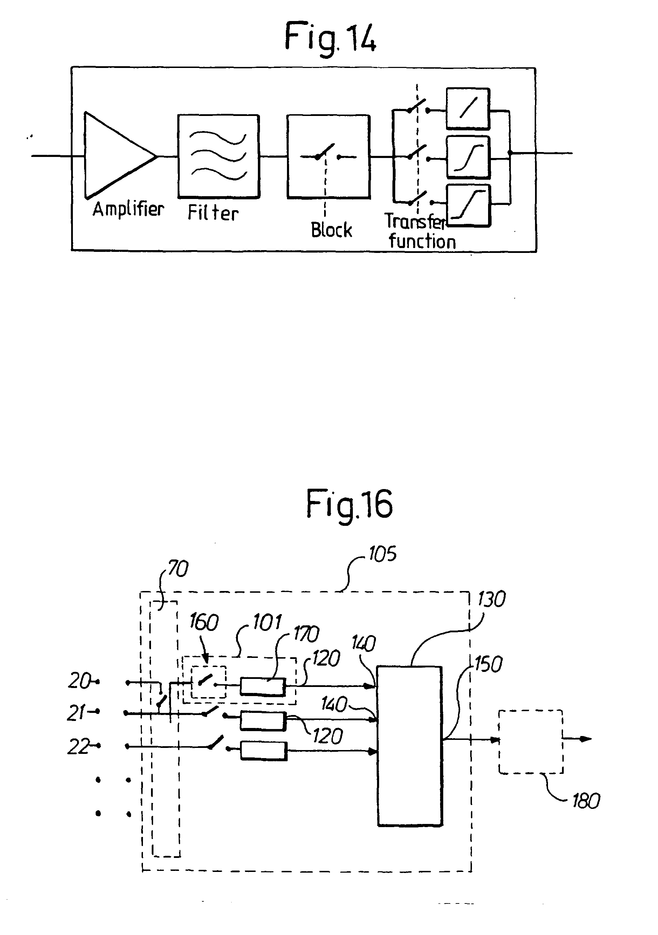

It may of course be possible to filter/transform the signals at a later stage of the processing of the signals in case there is a wish to do so. This is further discussed in connection with Fig. 13 and 14 below.

Fig. 6 shows the signal registered between the SVC (superior vena cava) and the CAN, and the signals registered between the TIP and the CAN. These signals may e.g. be processed in a neural network, the result of which is shown in Fig. 7, which shows the synthesized signals resulting from above processing. The signals in Fig. 6 are used as input signals to a neural network, and compared in practice with a range of a surface ECG (e.g. one heart beat interval) and compared in iterations with the weighted and processed input signals. Once the neural network NN is "trained" on the chosen interval the parameters of the NN are "frozen" to be used subsequently. The surface ECG used in the training of the network is that of the patient in this method, but not necessarily a surface-ECG taken at the same time but one of an earlier date.

In case there is an essential change in the appearance of the patients ECG a new surface-ECG-recording should take place and that recording should be used instead. The parameters are then adjusted in iterations to make the combined signal reassemble the new surface ECG and after a number of iterations in the chosen range the specific details of an ordinary ECG will evolve out of the information put into the neural network NN.

It should of course be clear that the method can, within the scope of the invention, include IEGMs recordings obtained from paired connected implanted electrodes such as SVC and Can, Tip and Can, RV and Can etc. All signals are according to the method obtained between two poles (groups of interconnected electrodes). Each group is either a single electrode: SVC, RV, Tip, Ring, Can or at least two electrically connected electrodes.

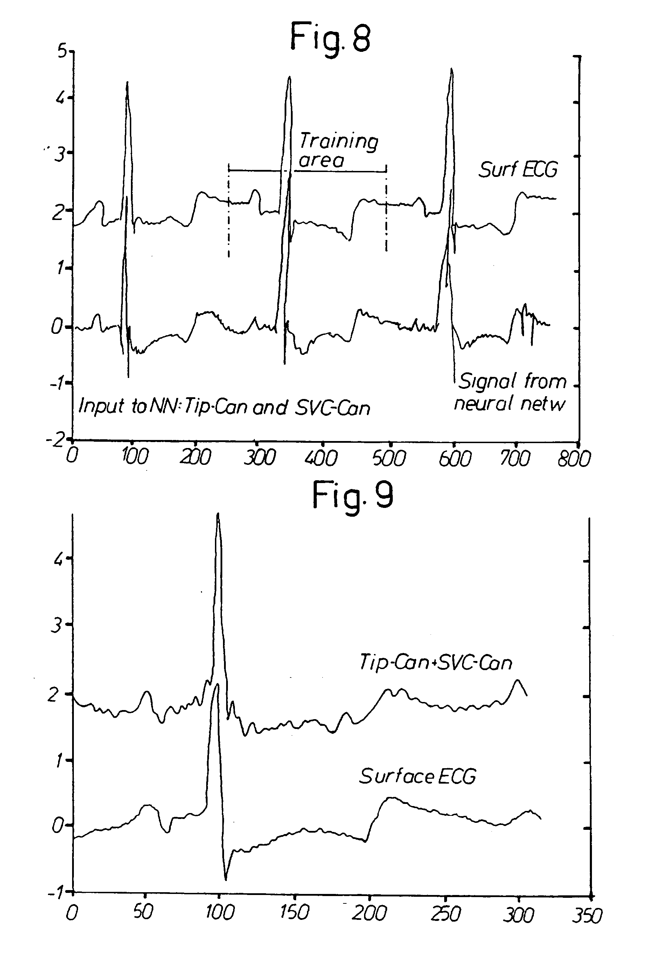

Fig. 8 shows an enlargement of the diagram in Fig. 7 showing the training area in greater detail. As is shown below in Fig. 9 the signals may also be added form combinations of interconnected electrodes.

Fig. 9 shows a) the signal resulting from measuring between unprocessed interconnected electrodes Tip(V) + Can and SVC(Superior Vena Cava) + Can after signal processing in accordance with the invention and below is found a surface ECG. One may note the close resemblance between the two signals. This is just one of many possible electrode combinations according to this invention.

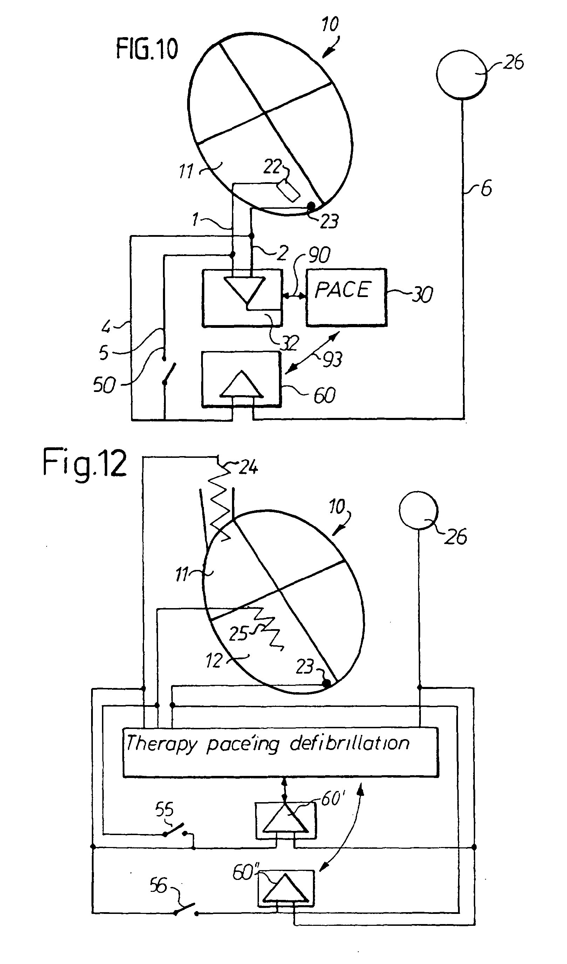

Fig. 10 shows a first embodiment of the invention comprising an implanted heart stimulator having two electrodes, a ring electrode 22 and a tip electrode 23 implanted in the right ventricle 11 of a heart 10. Conductors 1 and 2 from the electrodes 22 and 23, respectively, are connected to the input terminals on a ventricular monitoring unit 32. The output signal from the unit 32 will consist of a ventricular IEGM. The ventricular IEGM is then used e g for controlling the pacing executed by the active implant 30. This part of the active implanted system corresponds to the usual setup for pacing. Conductors 4 and 5 connected to the electrodes 23 and 22, respectively are also connected to the input side of an synthesizing unit 60. The conductors may be physically (electrically) connected to each other before the input terminal of the unit 60 or they may be processed in the unit 60 as will be discussed later. In one of the conductors 4 or 5 a switch 50 is arranged. Connection of the switch 50 and the action of unit 60 can be implemented in a multiplex mode. This is also true in the embodiments below. In the drawing is also shown a can electrode 26, which may be the active implant case. This electrode 26 is connected via a conductor 6 to the input side of the same unit 60.

According to the invention the signals received from the ring electrode 22 and the tip electrode 23 are combined before the synthesizing unit 60 according to the invention. The switch 50 may be controlled to either let the signal from the ring electrode 22 pass or not to the synthesizing unit 60. The signal measured at the can electrode 26 is also fed to this unit and combined with the signal from 22 and 23 and thus when switch 50 is closed this will provide the synthesized S-ECG according to the invention.

The reason for adding the signals from the electrodes 22 and 23 is that they will simulate a bigger electrode. This is of course true for all possible electrode combinations forming a group in the sense of this invention.

A connection between the synthesizing unit 60 and the active implant 30 in the form of a two-headed arrow 93 is shown implying the possibility to control the active implant in accordance with the information received from the synthesized ECG. Such an arrow 90 is also shown between the ventricular monitoring unit 32 and the active implant 30 to indicate the existing possibilities for controlling the active implant. However, the arrangement for stimulating the heart from the active implant is not shown explicitly in the drawing. This is well know technique within the art.

The embodiment depicted in Fig. 10 may of course include other electrodes implanted intra- and/or extracardially and also the combination of signals may be between two electrodes and the CAN as shown or between the CAN and 3 or 4 implanted electrodes, respectively. The minimum requirements is the combination of signals from two electrodes, and this does not necessarily include the CAN.

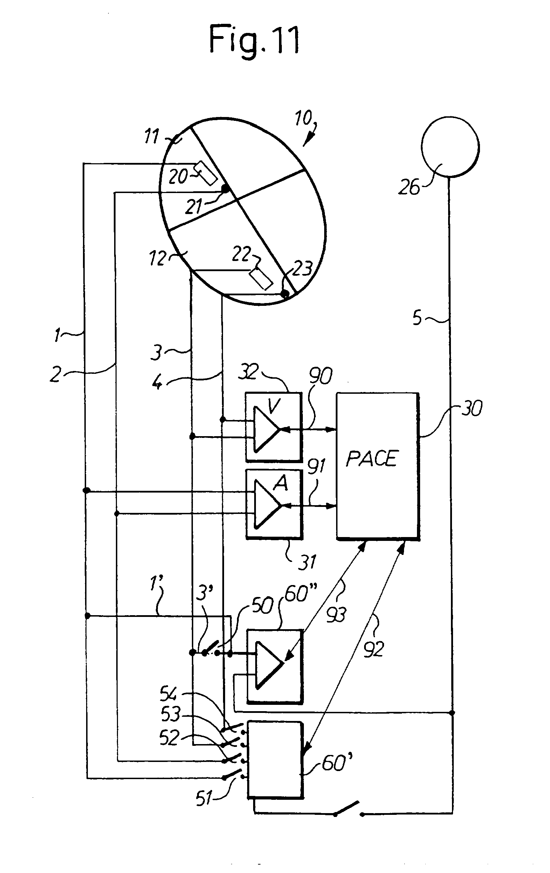

Another embodiment of the heart stimulation system including the invention is shown in Fig. 11 in which an active implant having four electrodes, a ring electrode 20 and a tip electrode 21 implanted in the right atrium 11 and a ring electrode 22 and a tip electrode 23 implanted in the right ventricle 12 of a heart 10 . The ring electrode 22 and the tip electrode 23 are connected via conductors 3 and 4, respectively, to the input of a ventricular monitoring unit 32. A ventricular IEGM may be obtained in the ventricular monitoring unit 32. The ring electrode 20 and the tip electrode 21 are connected via conductors 1 and 2 respectively to an atrial monitoring unit 31. An atrial IEGM may be obtained in the atrial monitoring unit 31. The obtained IEGMs are then used for controlling the pacing executed by the active implant 30, which is indicated in the drawing by two-headed arrows 90, 91. This part of the active implant system corresponds to the usual setup for pacing.

In the drawing is also shown a can electrode 26 , which may be the active implant case.

Fig. 11 shows how to process synthesized or interconnected ECGs in two synthesizing units 60' and 60". The intracardiac electrodes 20, 21, 22, 23 are also connected via conductors 1, 2, 3, 4 respectively to the input side of the unit 60'. Switches 51, 52, 53, 54 are provided in the conductors 1, 2, 3, 4 in order to connect or disconnect the electrodes 20, 21, 22, 23 to or from the input terminal of the unit 60'. To the input side of the same unit 60' the can electrode 26 is connected via conductor 5. The can electrode 26 is also connected via conductor 5 to the input terminal of the synthesizing unit 60" as are the two ring electrodes 20 and 22 via conductors 1' and 3', respectively. In the same manner as above a switch 50 is provided in the conductor 3'.

The unit 60' is a unit with a more sophisticated form of signal processing.

A connection in the form of two-headed arrows 92 and 93 between the synthesizing units 60' and 60", respectively, and the active implant 20 are shown so as to indicate the possibility to control the active implant in accordance with the information received from the synthesized ECGs.

Yet another embodiment is shown in Fig. 12. In the heart 10 is implanted in the superior vena cava a defibrillation electrode 24 and in the right ventricle 12 another defibrillation electrode 25 and a tip electrode 23 and the can 26. The electrodes are all connected via conductors to the active implant for pacing and defibrillation as is usual. Also shown is the possibility of obtaining an synthesized ECG in the atrial and ventricular monitoring units 60' and 60", respectively by the possibility of closing or opening switches 55 and/or 56. The difference from the other embodiments mainly lies in the use of the defibrillation electrodes in the application. This indicates that the active implant in this embodiment can be used for sensing of cardiac events, for pacing in a normal manner and for detection of anomalies in the behaviour of the heart which will necessitate defibrillation. The synthesized ECG may in this embodiment, as in the other possible embodiments according to this invention, provide information to be used by the active implant for providing specific actions depending on the behaviour of the heart.

Referring to Fig. 13 there is shown a schematic presentation of a heart 10, having a right atrium 11 and a right ventricle 12. A system comprising five intracardiac electrodes is shown implanted in the heart, in the atrium 11 a ring electrode 20, a tip electrode 21 and in the ventricle 12 a ring electrode 22, a tip electrode 23 and a defibrillation electrode 25. The system also comprises a patch electrode 27 in contact with the outside of the heart, a defibrillation electrode 24 is placed near where the superior vena cava 13 enters the right atrium, and also the can electrode 26.

Each of the above electrodes are connected to the input side of a transforming unit 101, resp., and the can electrode 26 is likewise connected to the input side of each one of the transforming units 101 as is indicated by 40. Shown is also a bus 41 connected to the outputs of each one of the units 101. The bus 41 is used for applying individual weights to each output signal 42a...42g in the unit 130 from which an output signal is generated forming a synthesized ECG.

Using the system presented schematically in Fig. 13 it is possible to register and/or process signals from two or more electrodes. The transforming units 101 in Fig. 13 is shown in greater detail in Fig. 14.

In these transforming units 101 the intracorporal signals from two or more electrodes, of which one electrode may be the can 26, will be amplified, filtered and possibly blocked during certain time intervals. This may be done by conventional means using well know techniques within the art, such as by using thresholds, or windows restricting either the amplitudes or the frequency of the signals received. The blocking of the signals may be individual at predetermined and controllable time intervals, such that the particular signal is prevented from affecting the result at the chosen time intervals. This signal processing will result in pretreated second signals

These individually processed second signals from two or more electrodes may then be summed together and once more signal processed in a second processing procedure according to the invention, either in the processing unit 130 or by other means.

Below the schematic picture of the heart 10 is shown a schematic drawing of the principle according to which the signal processing is carried out. First the received signals will pass through the filtering/transforming and blocking circuitry 101, comprising one or more amplifier, filter, block and transfer functions. The reason for this first processing is that the intracardiac electrodes will each pick up information as to the electrical activity resulting from cardiac activity in the proximity of the electrode. But they will not only pick up the information activities occurring in the proximity of the electrode but also from activity in other parts of the heart. The electrical activity induced in the very near proximity of each electrode will of course give a considerable greater contribution to the signal than activity occurring comparatively farther away.

The processed signals will then be fed to a second signal processing unit 130 for the summation including the weighting of the individual signals and processed to generate the synthesized surface ECG.

Electrodes having a large surface and positioned extracardially normally give signals better suited for synthesizing a surface ECG. The signals from intracardiac and extracardiac electrodes can be used for mathematical processing by signal processing. The signals will thus be processed in order to filter out e.g. high frequency components which mostly are the result of local (in the close proximity) electrical activity. This filtering effect can be accomplished in a more subtle fashion by blocking the signal during a given time period. Such a circuit can e.g. be realized by circuitry which detects the fast slope of the IEGM from a bypassing wavefront of electrical activity at an early stage, thereby blocking most of the locally produced signal.

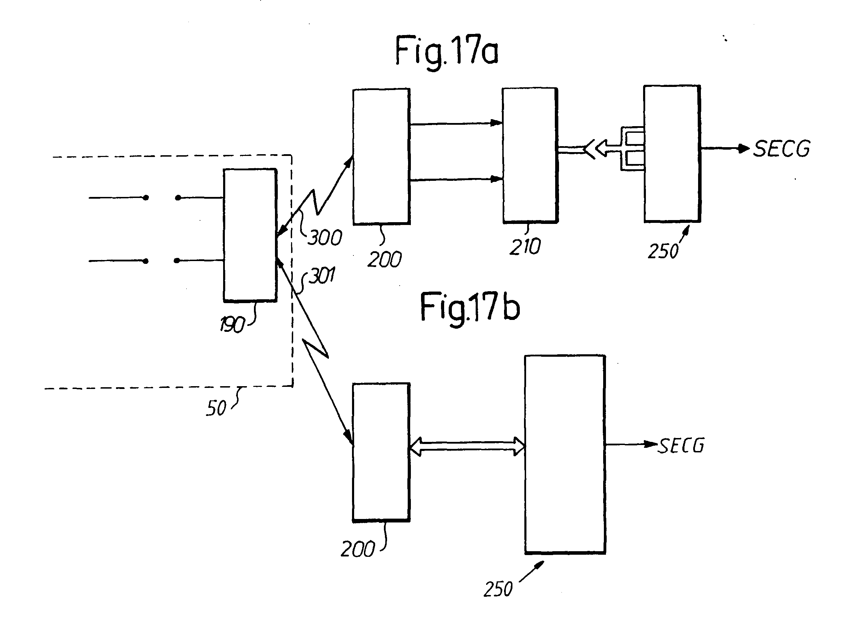

If a small delay in the actual viewing of the signals, i.e. the synthesized surface ECG, can be tolerated the result of the signal processing may be even more improved. If the signal first is stored then mathematical processing may be done with a slight time delay, which will give time and possibility to remove from the signal, the stored IEGM from the electrode, the part that originates locally, i.e. the contribution from electrical activity in the close proximity of the electrode. The part that is removed may then be substituted by interpolation. The stored signal will then according to the invention be used instead of the directly obtained signal for the filtering, blocking and multiplication by constants in the second processing procedure with the other intracoporal signals according to the Fig. 13. This is shown in Fig. 17a.

During the processing each signal will be multiplied by a certain constant. This is done in order to favour signals which contain more of far field ECG signals.

Still another improvement is the exaggerating of low amplitude signals like P-wave in comparison with a QRS-complex. This may be done in the block named "linear and non-linear transfer functions" in Fig. 13 and 14 and Fig. 5b. (conf. discussion in connection with Fig. 5b.) Amplitudes outside specified limits may be clipped or gradually limited by a sigmoidal transfer function.

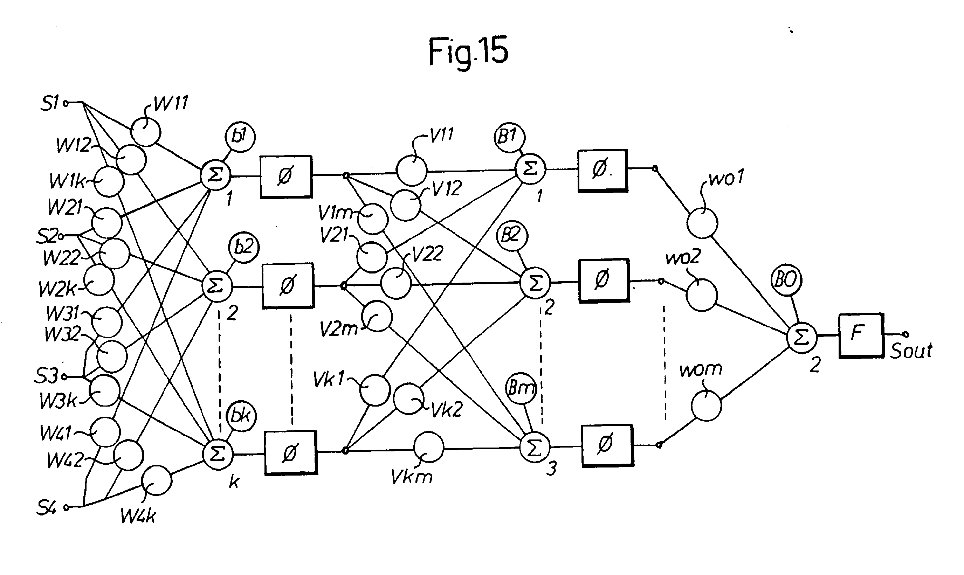

One way of implementing the signal processing is to use a neural network. The principle of such a technique will be shortly described here and with reference to Fig. 15. A thorough description of a neural network and its use is disclosed in the book "Neural Networks, a comprehensive foundation" by Simon Haykin, Macmillan College Publishing Company, ISBN 0-02-352761-7.

A neural network has mostly several input signals and one or more output signals. Each input signal is processed and combined with the other weighted input signals. The combination of the signals are further processed in order to produce one or more output signals. The processing of the signals contains multiplication, addition and non-linear signal handling. A neural network may have different structures. This structure must first be settled. The neural network then has a number of unknown parameters (weights and biases). Characteristic for the neural network is that it must learn how to adjust these parameters. The learning action is performed on short, but sufficiently long and representative input signal intervals which are fed into the neural network in order to produce an output signal, which will be compared with a reference signal. The learning procedure is performed by repeatedly feeding these input signals to the neural network and adjusting each parameter so that finally the output signal from the neural network resembles the reference signal. The neural network is now adjusted. New input signals will then produce output signals, which will be similar to the signals that the process, which the neural network is simulating, will produce.

The interior of the neural network will now be described in conjunction with Fig. 15. The input signals are S1, S2, S3 and S4. Each input signal is then multiplied by a certain weight (w11 to w4k). For every input signal there is so may weights as there are summing junctions. In figure 15 there are k summing junctions. In the summing junction all weighted signals and a bias signal are added. The bias signal is just a constant voltage.

The output signal from the summing junctions is then passing a transfer function, denoted "Φ". Very often this transfer function is a non-linear function, like the sigmoid function. In the very same way as the input signals are processed the output signals from the transfer function blocks are now handled. Here there are new weights, v11 to vkm, B1 to Bm. Fig. 16 shows only a single output, Sout. The transfer function is denoted "*F*". Normally this last transfer function is a linear function.

In the invention Sout is the synthesized surface ECG and the input signals are the intracardiac and/or intrathoracic signals, which contains information about the electrical activity of the heart.

A similar way to implement this invention is to use Fuzzy Logic instead of a neural network. There are similarities between neural network and fuzzy logic. Because of this reason software exists that can transform a neural network into an electronic circuit utilizing the Fuzzy Logic technique.

The invention may be implemented in several ways. The signal processing may take place in a device incorporated in the active implant as an integral part of the same or it may take place out of the active implant. The active implant may also comprise a transmitter means for transmitting the synthesized ECG via telemetry.

It is to be understood that transmitters mentioned in the description may for reasons of communication by telemetry be a combined transmitter/receiver, or the type of telemetric unit usually provided in active implants.

Fig. 16 shows the outputs from the implantable electrodes 20,21,22.... and to each electrode the corresponding inputs to a signal processing device 105. The signal processing device 105 comprises controllable switching means 160, transforming means 170 in a first unit 101, with one such unit for each input. Each first unit may also comprise e.g. amplification means. Each first unit 101 is also provided with an output 120. A second signal processing unit 130 is also shown having inputs 140 and an output 150. Several outputs may of course be provided. Connected to the signal processing device 105 is a transmitter 180 for transmitting the signals processed.

In the device is also provided switching means schematically shown at 70 for selectively interconnection of the input signals from one or more electrodes before the signal processing units 101. These switches may be connected/disconnected in a multiplexing mode in order to facilitate the process in the active implant, since when short circuiting two or more of the intracardiac electrodes this will affect the pacing/stimulating arrangement in the active implant in which the device according to the invention or part of the device according to the invention is included.

In Fig. 17 is shown another embodiment of the device according to the invention in which there is provided outputs from the implantable electrodes. A transmitter 190 for receiving the signals from the electrodes is also arranged. This part of the device is placed in an active implant 50.

The transmitter 190 is arranged to transmit the signals from the implantable electrodes and two pathways 300 and 301 are shown in order to illustrate two alternative embodiments of the device according to the invention. Actually there need only be one pathway in each embodiment. After the pathway is shown a receiver and in the first alternative Fig. 17a a memory unit 210 for storing of the signals to be processed later is supplied. The memory unit 210 is connectable via a data bus to a processing unit 250. It should be understood in this document that a radio receiver and a radio transmitter are merely examples of a type of receiver and a transmitter. The signals could just as well be transmitted by any method which is well know with in the art.

In the second embodiment shown in Fig. 17b the receiver 200 is connected to a signal processing device 250 according to the invention.

In these three last alternative embodiments of the invention it is of course possible to send the unprocessed signals or the processed signals at one stage or the other via the public telephone network to the place where the synthesized ECG -signal may be viewed and evaluated.

The memory unit for storing may also of course be situated in the active implant. The active implant will of course also comprise components usually provided in an active implant such as pacing and /or defibrillation circuitry etc.

Although the invention has been described with respect to particular embodiments, it is to be understood that these embodiments are merely illustrative to the application of the principles of the invention. Numerous modifications may be made therein and other arrangements may be devised without departing from the scope of the invention.

## Claims

1. An extracorporal device (200, 250) for performing follow up procedures for patients having an implanted cardiac pulse generator (50),
said implanted pulse generator having means for sensing IECG signals originating from said patient's heart,
said implanted cardiac pulse generator having a transmitter (190) for transmitting IECG signals to said extracorporal device,
said extracorporal device having a telemetry receiver (200) for receiving IECG signals from said implanted pulsegenerator wherein
said extracorporal device comprises signal processing means (250) for transforming said IECG signals to a synthesized surface ECG signal,
said extracorporal device comprising means for using said synthesized surface ECG equivalent signal during said follow up procedure.

2. An implantable cardiac pulse generator having means (20, 21, 22) for sensing IECG signals originating from a patient's heart wherein
said implantable cardiac pulse generator comprises a signal processing means (105) for transforming said IECG signals to a synthesized surface ECG signal,
said implantable pulse generator further comprising a transmitter for transmitting said synthesized surface ECG to an extracorporal device.

3. An extracorporal device according to claim 1 or an implantable pulse generator according to claim 2 **characterized in that** it comprises a signal processing device having a plurality of first individual signal processing units (101), each first unit (101) having an input (110) for receiving a first input signal, relating to said first signal being intracorporally sensed heart activity, and an output (120);
- a second signal processing unit (130) having a plurality of inputs (140), each input (140) being coupled to one of said outputs (120) of said first units, said second signal processing unit having at least one output (150);
- each of said first signal units (101) being set up to treat at least one input signal each in accordance with at least one selected transfer function, such that it produces a pretreated signal as its output (120);
- said second signal processing unit (130) being set up to combine at least two of said pretreated signals such that at least one synthesized ECG-signal (SECG) is generated and provided at said at least one output (150).

4. The signal processing device according to claim 3, comprising a plurality of switching means (70) for selectively connecting predetermined combinations of said first input signals.

5. The signal processing device according to any of the claims 3 to 4, wherein each said first unit (101) comprises a controllable switch means (160) for individually blocking the respective input signal during selected time intervals.

6. The signal processing device according to any of the claims 3 to 5, wherein each said first unit (101) comprises a transforming unit (170), said transforming unit transforming said input signal in accordance with said at least one selected transfer function.

7. The signal processing device according to any of the claims 3 to 6, wherein said at least one selected transfer function is selected from a group consisting of a linear, a limited and/ or a sigmoidal transfer function.

8. The signal processing device according to any of the claims 3 to 7, wherein said first input signals originates from at least two implantable electrodes (20, 21, 22, 23, 24, 25, 26, 27).

9. A signal processing device according to any of the claims 3-8 wherein said means for combining at least two of said signals is an assosiative component type neural network, fuzzy logic and/or summation.

10. A signal processing device according the claim 9 wherein said neural network operates in a manually trained mode.

11. A signal processing device according the claim 9 wherein said neural network operates in a self-learning mode.

12. A method for synthesizing at least one surface ECG signal wherein:
- sensing of a plurality of first signals from implanted intra- and extracardial electrodes (20, 21, 22, 23, 24, 25, 26, 27), said first signals measured between any of two of said electrodes comprising information relating to events in the close proximity of the respective electrodes and to events not in the proximity
- treating said signals by blocking the signals during chosen time intervals relating to specific evoked or intrinsic activity in the heart
- producing second signals by processing said first signals with at least one selected transfer function
- combining at least two groups of signals, each of said at least two groups comprising at least one of said second signals, to produce combined signals
- processing said at least two combined signals in a second processing procedure to produce at least one synthesized surface ECG-signal.

13. A method in accordance with claim 12 wherein said first and/or second signals are individually blocked during selected time intervals.

14. A method in accordance with claim 12 or 13 wherein said at least one selected transfer function is selected from a group consisting of a linear, a limited and/or a sigmoidal transfer function.

15. A method according to any of the claims 12 to 14 wherein said summation process comprises the processing of the combined signals from said at least two groups of signals, each of which comprises at least one of said second signals.

16. A method according to any of the claims 12 to 15 wherein said summation process is accomplished by an operation of an associative component type neural network and/or fuzzy logic and/or summation.

17. A method according to any of the claims 12 to 16 wherein a surface ECG from a person, from whom the input signals relating to heart activity stems, is used as input reference signal to said neural network during one or more training procedures.

18. A method according to claims 16 to 17 wherein said neural network operates in a manually trained mode.

19. A method according to claims 16 to 17 wherein said neural network operates in a self-learning mode.

## Patentansprüche

1. Extrakorporale Vorrichtung (200, 250) zur Durchführung von Nachfolgebehandlungen bei Patienten mit einem implantierten Herzimpulsgenerator (50),
wobei der genannte implantierte Impulsgenerator eine Vorrichtung zum Abfühlen von IECG-Signalen aufweist, die von dem genannten Herz des Patienten herrühren,
der genannte implantierte Herzimpulsgenerator einen Sender (190) zum Aussenden von IECG-Signalen zu der genannten extrakorporalen Vorrichtung aufweist,
die genannte extrakorporale Vorrichtung einen Telemetrieempfänger (200) zum Empfangen von IECG-Signalen von dem genannten implantierten Impulsgenerator aufweist, wobei die genannte extrakorporale Vorrichtung eine Signalverarbeitungsvorrichtung (250) zum Umwandeln der genannten IECG-Signale in ein synthetisiertes Oberflächen ECG-Signal enthält,
die genannte extrakorporale Vorrichtung Mittel zur Verwendung des genannten äquivalenten synthetisierten Oberflächen ECG- Signals während der genannten Nachfolgebehandlung enthält.

2. Implantierbarer Herzimpulsgenerator mit einer Vorrichtung (20, 21, 22) zum Abfühlen von aus dem Herzen eines Patienten stammenden IECG-Signalen, wobei der genannte implantierbare Herzimpulsgenerator eine Signalverarbeitungsvorrichtung (250) zum Umwandeln der genannten IECG-Signale in ein synthetisiertes Oberflächen ECG-Signal enthält,
der genannte implantierbare Impulsgenerator ferner einen Sender zum Senden des genannten synthetisierten Oberflächen ECG zu einer extrakorporalen Vorrichtung enthält.

3. Extrakorporale Vorrichtung nach Anspruch 1 bzw. implantierbarer Impulsgenerator nach Anspruch 2, **dadurch gekennzeichnet, dass** sie bzw. er eine Signalverarbeitungsvorrichtung mit einer Mehrzahl von ersten individuellen Signalverarbeitungseinheiten (101) enthält, wobei jede erste Einheit (101) einen Eingang (110) für den Empfang eines ersten Eingangssignals aufweist, das sich auf das genannte erste Signal bezieht, welches die intrakorporal abgefühlte Herzaktivität darstellt, und einen Ausgang (120);
- eine zweite Signalverarbeitungseinheit (130) mit einer Mehrzahl von Eingängen (140) enthält, wobei jeder Eingang (140) mit einem der genannten Ausgänge (120) der genannten ersten Einheiten gekoppelt ist, die genannte zweite Signalverarbeitungseinheit wenigstens einen Ausgang (150) aufweist;
- jede der genannten ersten Signaleinheiten (101) ausgebildet ist, wenigstens ein Eingangssignal jeweils in Übereinstimmung mit wenigstens einer ausgewählten Übertragungsfunktion so zu behandeln, dass sie als Ausgang (120) ein vorbehandeltes Signal erzeugt;
- die genannte zweite Signalverarbeitungseinheit (130) ausgebildet ist, wenigstens zwei der genannten vorbehandelten Signale so zu kombinieren, dass wenigstens ein synthetisiertes ECG-Signal (SECG) erzeugt und an dem genannten wenigstens einen Ausgang (150) geliefert wird.

4. Signalverarbeitungsvorrichtung nach Anspruch 3, enthaltend eine Vielzahl von Schaltvorrichtung (70) zum selektiven Verbinden vorbestimmter Kombinationen der genannten ersten Eingangssignale.

5. Signalverarbeitungsvorrichtung nach einem der Ansprüche 3 bis 4, wobei jede der genannten ersten Einheiten (101) eine steuerbare Schaltvorrichtung (160) zum individuellen Blockieren des betreffenden Eingangssignals während ausgewählter Zeitintervalle enthält.

6. Signalverarbeitungsvorrichtung nach einem der Ansprüche 3 bis 5, wobei jede der genannten ersten Einheiten (101) eine Umwandlungseinheit (170) enthält, die genannte Umwandlungseinheit das genannte Eingangssignal in Übereinstimmung mit der genannten wenigstens einen ausgewählten Übertragungsfunktion umwandelt.

7. Signalverarbeitungsvorrichtung nach einem der Ansprüche 3 bis 6, wobei die genannte wenigstens eine ausgewählte Übertragungsfunktion aus einer Gruppe ausgewählt ist, die aus einer linearen, einer begrenzten und/oder einer Sigmoidal-Übertragungsfunktion besteht.

8. Signalverarbeitungsvorrichtung nach einem der Ansprüche 3 bis 7, wobei die genannten ersten Eingangssignale von wenigstens zwei implantierbaren Elektroden (20, 21, 22, 23, 24, 25, 26, 27) stammen.

9. Signalverarbeitungsvorrichtung nach einem der Ansprüche 3 bis 8, wobei die genannte Vorrichtung zum Kombinieren von wenigstens zwei der genannten Signale ein neurales Netzwerk vom assoziativen Komponententyp, eine Fuzzi-Logik und/oder eine Summation ist.

10. Signalverarbeitungsvorrichtung nach Anspruch 9, wobei das genannte neurale Netzwerk in einem manuell trainierten Modus arbeitet.

11. Signalverarbeitungsvorrichtung nach Anspruch 9, wobei das genannte neurale Netzwerk in einem Selbstlernmodus arbeitet.

12. Verfahren zum Synthetisieren wenigstens eines Oberflächen ECG-Signals, mit:
- Abfühlen einer Mehrzahl von ersten Signalen von implantierten intra- und extrakardialen Elektroden (20, 21, 22, 23, 24, 25, 26, 27), wobei die genannten ersten Signale zwischen irgendwelchen von zwei der genannten Elektroden gemessen werden, die Informationen enthalten, welche sich auf Ereignisse in enger Nachbarschaft zu den betreffenden Elektroden und auf Ereignissen nicht in der Nachbarschaft beziehen,
- Behandeln der genannten Signale durch Blockieren der Signale während ausgewählter Zeitintervalle, die sich auf eine spezielle evozierte oder intrinsische Aktivität im Herzen beziehen,
- Erzeugen zweiter Signale durch Verarbeiten der genannten ersten Signale mit wenigstens einer ausgewählten Übertragungsfunktion,
- Kombinieren wenigstens zweier Gruppen von Signalen, wobei jede der genannten wenigstens zwei Gruppen wenigstens eines der genannten zweiten Signale enthält, um kombinierte Signale zu erzeugen,
- Verarbeiten der genannten wenigstens zwei kombinierten Signale in einer zweiten Verarbeitungsprozedur, um wenigstens ein synthetisiertes Oberflächen ECG-Signal zu erzeugen.

13. Verfahren nach Anspruch 12, wobei die genannten ersten und/oder zweiten Signale während ausgewählter Zeitintervalle individuell blockiert werden.

14. Verfahren nach Anspruch 12 oder 13, wobei die genannte wenigstens eine ausgewählte Übertragungsfunktion ausgewählt ist aus einer Gruppe bestehend aus einer linearen, einer begrenzten und/oder einer Sigmoidal-Übertragungsfunktion.

15. Verfahren nach einem der Ansprüche 12 bis 14, wobei der genannte Summationsprozess die Verarbeitung der kombinierten Signale aus den genannten wenigstens zwei Gruppen von Signalen enthält, von denen jede wenigstens eines der genannten zweiten Signale enthält.

16. Verfahren nach einem der Ansprüche 12 bis 15, wobei der Summierungsprozess durch die Operation eines neuralen Netzwerkes des assoziativen Komponententyps und/oder eine Fuzzi-Logik und/oder eine Summation ausgeführt wird.

17. Verfahren nach einem der Ansprüche 12 bis 16, wobei ein Oberflächen ECG von einer Person, von der die sich auf die Herzaktivität beziehenden Eingangssignale stammen, als Eingangsbezugssignal in das neurale Netzwerk verwendet wird, während einer oder mehrere Trainingsprozeduren.

18. Verfahren nach den Ansprüchen 16 bis 17, wobei das genannte neurale Netzwerk in einem manuell trainierten Modus arbeitet.

19. Verfahren nach den Ansprüchen 16 bis 17, wobei das genannte neurale Netzwerk in einem Selbstlernmodus arbeitet.

## Revendications

1. Dispositif (200, 250) extracorporel pour effectuer des procédures de suivi de patients ayant un générateur (50) implanté d'impulsions cardiaques,
le générateur implanté d'impulsions ayant des moyens de détection de signaux d'électrogrammes intracardiaques provenant du coeur du patient,
le générateur implanté d'impulsions cardiaques ayant un émetteur (190) pour transmettre des signaux d'électrogrammes intracardiaques au dispositif extracorporel,
le dispositif extracorporel ayant un récepteur (200) de télémétrie pour recevoir des signaux d'électrogrammes intracardiaques du générateur implanté d'impulsions, dans lequel
le dispositif extracorporel comprend des moyens (250) de traitement du signal pour transformer les signaux d'électrogrammes intracardiaques en un signal d'électrocardiogramme de surface synthétisé,
le dispositif extracorporel comprenant des moyens pour utiliser le signal équivalent d'électrocardiogramme de surface synthétisé pendant l'opération de suivi.

2. Générateur implantable d'impulsions cardiaques ayant des moyens (20, 21, 22) de détection de signaux d'électrogrammes intracardiaques provenant d'un coeur de patient, dans lequel
le générateur implantable d'impulsions cardiaques comprend des moyens (105) de traitement du signal pour transformer les signaux d'électrogrammes intracardiaques en un signal d'électrocardiogramme de surface synthétisé,
le générateur d'impulsions implantable comprenant, en outre, un émetteur pour envoyer l'électrocardiogramme de surface synthétisé à un dispositif extracorporel.

3. Dispositif extracorporel suivant la revendication 1 ou générateur implantable d'impulsions suivant la revendication 2, **caractérisé en ce qu'**il comprend un dispositif de traitement du signal ayant :
- une pluralité de premières unités (101) individuelles de traitement du signal, chaque première unité (101) ayant une entrée (110) de réception d'un premier signal d'entrée se rapportant au premier signal qui est de l'activité cardiaque détectée intracorporellement, et une sortie (120) ;
- une deuxième unité (130) de traitement du signal ayant une pluralité d'entrées (140), chaque entrée (140) étant couplée à l'une des sorties (120) des premières unités, la deuxième unité de traitement du signal ayant au moins une sortie (150) ;
- chacune des premières unités (101) de signal étant réglée pour traiter au moins un signal d'entrée en fonction d'au moins une fonction de transfert sélectionnée de façon à produire un signal prétraité à sa sortie (120) ;
- la deuxième unité (130) de traitement du signal étant réglée pour combiner au moins deux des signaux prétraités de façon à produire au moins un signal d'électrocardiogramme synthétisé (SECG) et à l'envoyer à ladite au moins une sortie (150).

4. Dispositif de traitement du signal suivant la revendication 3 comprenant une pluralité de moyens (70) de commutation pour connecter sélectivement des combinaisons déterminées à l'avance de premiers signaux d'entrée.

5. Dispositif de traitement du signal suivant l'une quelconque des revendications 3 à 4, dans lequel chaque première unité (101) comprend des moyens (160) de commutation qui peuvent être commandés pour arrêter individuellement le signal d'entrée respectif pendant des intervalles de temps sélectionnés.

6. Dispositif de traitement du signal suivant l'une quelconque des revendications 3 à 5, dans lequel chaque première unité (101) comprend une unité (170) de transformation, l'unité de transformation transformant le signal d'entrée en fonction de la au moins une fonction de transfert sélectionnée.

7. Dispositif de traitement du signal suivant l'une quelconque des revendications 3 à 6, dans lequel la au moins une fonction de transfert sélectionnée est sélectionnée dans un groupe consistant en une fonction de transfert linéaire, limitée et/ou sigmoïde.

8. Dispositif de traitement du signal suivant l'une quelconque des revendications 3 à 7, dans lequel les premiers signaux d'entrée proviennent d'au moins deux électrodes (20, 21, 22, 23, 24, 25, 26, 27) implantables.

9. Dispositif de traitement du signal suivant l'une quelconque des revendications 3 à 8, dans lequel les moyens de combinaison d'au moins deux des signaux sont un composant associatif de type réseau neuronal, logique flou et/ou sommation.

10. Dispositif de traitement du signal suivant la revendication 9, dans lequel le réseau neuronal fonctionne dans un mode à apprentissage manuel.

11. Dispositif de traitement du signal suivant la revendication 9, dans lequel le réseau neuronal fonctionne dans un mode d'auto-apprentissage.

12. Procédé de synthèse d'au moins un signal d'électrocardiogramme de surface, dans lequel :
- on détecte une pluralité de premiers signaux provenant d'électrodes (20, 21, 22, 23, 24, 25, 26, 27) implantées intracardiales et extracardiales, les premiers signaux mesurés entre n'importe lesquelles de deux des électrodes comprenant de l'information se rapportant à des évidements ayant lieu à proximité immédiate des électrodes respectives et à des évidements qui ne sont pas à cette proximité ;
- on traite les signaux en arrêtant les signaux pendant des intervalles de temps sélectionnés se rapportant à l'activité évoquée spécifique ou intrinsèque du coeur ;
- on produit des deuxièmes signaux en traitant les premiers signaux par au moins une fonction de transfert sélectionnée ;
- on combine au moins deux groupes de signaux, chacun des au moins deux groupes comprenant au moins l'un des deuxièmes signaux pour produire des signaux combinés ;
- on traite les au moins deux signaux combinés dans un deuxième processus de traitement pour produire au moins un signal d'électrocardiogramme de surface synthétisé.

13. Procédé suivant la revendication 12, dans lequel on arrête individuellement les premiers et/ou deuxièmes signaux pendant des intervalles de temps sélectionnés.

14. Procédé suivant la revendication 12 ou 13, dans lequel on choisit la au moins une fonction de transfert sélectionnée dans un groupe consistant en une fonction de transfert linéaire, limitée et/ou sigmoïde.

15. Procédé suivant l'une quelconque des revendications 12 à 14, dans lequel l'opération de sommation comprend le traitement des signaux combinés provenant des au moins deux groupes de signaux, chacun d'entre eux comprenant au moins l'un des deuxièmes signaux.

16. Procédé suivant l'une quelconque des revendications 12 à 15, dans lequel l'opération de sommation s'effectue en faisant fonctionner un composant associatif du type réseau neuronal et/ou logique flou et/ou sommation.

17. Procédé suivant l'une quelconque des revendications 12 à 16, dans lequel un électrocardiogramme de surface d'une personne dont les signaux d'entrée se rapportent à des troncs d'activité cardiaque est utilisé comme signal de référence d'entrée envoyé au réseau neuronal pendant une ou plusieurs procédures d'apprentissage.

18. Procédé suivant les revendications 16 à 17, dans lequel le réseau neuronal fonctionne dans un mode d'apprentissage manuel.

19. Procédé suivant les revendications 16 à 17, dans lequel le réseau neuronal fonctionne dans un mode d'auto-apprentissage.
